(19) Europäisches Patentamt
European Patent Office
Office européen des brevets

(11) Publication number: **0 124 160 B1**

(12) **EUROPEAN PATENT SPECIFICATION**

(45) Date of publication of the patent specification: **17.09.86**

(51) Int. Cl.⁴: **C 07 C 51/12**, C 07 C 53/08, C 07 C 69/14, C 07 C 67/36, B 01 J 23/44, B 01 J 27/24

(21) Application number: **84200479.8**

(22) Date of filing: **04.04.84**

(54) **A process for the preparation of carboxylic acids and/or esters.**

(30) Priority: **02.05.83 NL 8301535**

(43) Date of publication of application: **07.11.84 Bulletin 84/45**

(45) Publication of the grant of the patent: **17.09.86 Bulletin 86/38**

(84) Designated Contracting States: **BE DE FR GB NL**

(56) References cited:
**EP-A-0 018 927**
**EP-A-0 035 860**
**EP-A-0 085 204**
**EP-A-0 090 443**
**EP-A-0 108 437**
**GB-A-760 057**

(73) Proprietor: **SHELL INTERNATIONALE RESEARCH MAATSCHAPPIJ B.V., Carel van Bylandtlaan 30, NL-2596 HR Den Haag (NL)**

(72) Inventor: **Drent, Eit, Badhuisweg 3, NL-1031 CM Amsterdam (NL)**

(74) Representative: **Aalbers, Onno et al, P.O. Box 302, NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

**Description**

The invention relates to a process for the preparation of carboxylic acids and/or esters by carbonylating alcohols in the presence of a palladium-containing catalyst, a bromide or iodide source and an organo-nitrogen compound. The invention relates in particular to the preparation of acetic acid and/or methyl acetate by carbonylating methanol.

It is known from German patent specification 1,115,234 that acetic acid or an acetic acid and methyl acetate mixture can be obtained by reacting methanol with carbon monoxide in the presence of a cobalt catalyst and an iodine compound. However, said process has the drawback that a rather high reaction temperature (230°C) and a high pressure (642 bar) must be applied. A process in which the reaction proceeds at lower temperatures and pressures by using a rhodium catalyst in the presence of an iodine compound is described in UK patent specification 1,233,121. Rhodium is, however, very expensive and there is a need for a process in which practical results are achieved with cheaper catalysts.

Dutch patent application 6804847 (publication 149,481) describes the preparation of carboxylic acids by reacting alcohols with carbon monoxide at a temperature of at least 50°C in the presence of an iridium, platinum, palladium, osmium and/or ruthenium-containing catalyst and bromine or iodine or a bromine or iodine compound as promoter. The catalyst may optionally contain an amine, phosphine, arsine or stibine ligand. It is stated that ligands such as phosphines, arsines or stibines may be present in excess amounts in the reaction mixture. Of the said metals, palladium and ruthenium are cheaper than rhodium. However, the rate at which carboxylic acids are formed in the process according to Dutch patent application 6804847, particularly when the last-mentioned metals are used as catalyst, is far from perfect.

The rate at which the carboxylic acid is formed can be expressed as the number of grams of carboxylic acid formed per gram catalyst metal per gram bromine or iodine per hour. This method of expression is particularly apt because the reaction rate is generally in direct proportion to both the quantity of catalyst metal and to the quantity of bromine or iodine present in the bonded or non-bonded state in the reaction mixture. Should a noble-metal catalyst be used, it is desirable that, on account of the high price of the noble metal, the quantity of carboxylic acid formed per gram metal per hour be as large as possible. Furthermore, it is important that the carboxylic acid be formed in the presence of as little bromine or iodine or bromine or iodine compound as possible. Not only do bromine and iodine or compounds thereof such as, for example, methyl iodine often have a corrosive effect but they are also somewhat volatile, so that by distillation of the reaction product they are drawn out of the reactor and have to be recycled to the reactor. When a process is put into practice on a large scale, it is, for economic reasons, desirable to keep the quantity of material to be recycled as low as possible.

In most of the examples in the aforesaid Dutch patent application 6804847, use is made of a catalyst containing the expensive metal iridium (iridium being about as expensive as rhodium). As has been pointed out hereinbefore, the formation rate of the carboxylic acid is relatively low; in Example 2, it amounts to 2.8 g acetic acid/g Ir/g I/h at a temperature of 175°C. In Examples 24 and 26, where the cheaper metals palladium and ruthenium are used respectively, the rate at which the acetic acid is formed at 175°C amounts to about 0.15 g/g Pd/g I/h and about 0.9 g/g Ru/g I/h respectively.

US patent specification 3,856,856 describes a process for the preparation of carboxylic acids by reacting alcohols with carbon monoxide in the presence of a catalyst primarily consisting of cobalt iodide to which a platinum compound has been added as promoter. However, said process has the drawback that even after long reaction times only a highly incomplete conversion of the alcohol is attained. According to Example I, methanol conversion after 18 hours amounts to approx. 60%. Palladium and ruthenium are stated to be unsuitable as promoters.

The European patent published application EP-A-90443 describes a process for the preparation of acetic acid and/or methyl acetate by reacting methanol with carbon monoxide in the presence of a catalytic quantity of a soluble Group VIII metal compound, in particular palladium, a promoter RZ in which R represents a $C_1$-$C_3$ alkyl group or H and Z is chlorine, bromine or iodine, an iodide or a carboxylate of a Group IA or IIA metal or of a transition metal, and one or more ligands containing at least one electron-rich nitrogen atom. In the examples, 2-amino-pyridine, 2,6-pyridine disulphonic acid, N,N,N',N'-tetramethyl-ethylenediamine and 2,2'-bipyridine are used as ligands. In the descriptive part, pyrrolidone is stated to be a usable ligand. However, the quantities used are small, mostly in the order of about 0.01 mol per mol alcohol or less. The rate at which acetic acid is formed in this process is relatively low, amounting to, for instance, about 14 g/g Pd/g I/hour in test 6, Table II, at a temperature of 182°C and a pressure of 114 bar. In Table III tests are described that were performed at the very high temperature of 300°C. Recalculated to a temperature of 180°C, the reaction velocity of those tests is even lower than that of the Table II tests.

The present invention concerns a process in which carboxylic acids are formed at a higher rate, in terms of g carboxylic acid/g Pd/g or Br/h, at low pressures and using the cheaper noble metal palladium than is the case with processes known hitherto. According to the invention, carboxylic acids and/or esters are prepared by allowing an alcohol to react with carbon monoxide in the presence of a homogeneous palladium-containing catalyst, an iodide and/or bromide source and an organo-nitrogen compound. The process is characterized by the use of an organo-nitrogen compound containing an 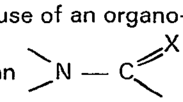 group (X being oxygen or sulphur) as promoter in a quantity of at least 0.3 mol per mol alcohol.

A process for the preparation of monocarboxylic acids by carbonylating alcohols in the presence of a nickel catalyst and the simultaneous presence of an alkali metal or alkaline earth metal halide, an alkyl halide and a solvent which may be an amide such as N-methylpyrrolidone is described in European patent specification 18927. However, the rate at which the carboxylic acid is formed is rather low. With the process according to the invention, in which, as shall hereinafter be explained, a nickel compound may optionally be used as co-catalyst, far higher rates of formation of, for instance, 25 g carboxylic acid/g Ni/g l/hour are readily achieved, also in relation to the quantity of nickel present.

In the process according to the invention, a carboxylic acid is initially produced containing one carbon atom more than the starting alcohol. The carboxylic acid formed reacts with non-converted alcohol to form an ester, so that a carboxylic acid and ester mixture is produced, the composition of which depends, inter alia, on the equilibrium position of the esterization reaction.

The organo-nitrogen compound acting as promoter, which contains an 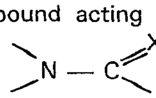 group, should form a solution with the reactants and/or products formed under the reaction conditions applied. Amides, carbamates and urea (derivatives) that are liquid at the temperature and pressure applied in the course of the reaction are highly suitable. Usable amides have, for example, the formula

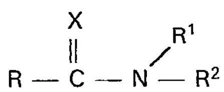 I

in which R represents a hydrogen atom or an alkyl, cycloalkyl, aryl, alkaryl or aralkyl group with not more than 10 carbon atoms, $R^1$ and $R^2$ represent, independently of each other, a hydrogen atom or an alkyl, aryl, alkaryl or aralkyl group, which may contain a $-\overset{X}{\overset{\|}{C}}$-group, with not more than 10 carbon atoms or $R^1$ and $R^2$ together with the nitrogen atom to which they are bonded form a cyclic group with not more than 5 carbon atoms, which may contain one or more nitrogen or oxygen atoms, or one of the groups $R^1$ and $R^2$ together with the nitrogen atom and R forms a cyclic group with not more than 5 carbon atoms, and X represents oxygen or sulphur. Specific examples of such amides are formamide, acetamide, N-methylacetamide, N-ethylacetamide, N,N-dimethylformamide, N,N-diethylformamide, N,N-dimethylacetamide, N,N-diethylacetamide, N-methyl-N-ethyl-acetamide, N,N-dimethylcyclohexylcarboxamide, N,N-dimethylbenzamide, N-phenylacetamide, N,N-diphenylacetamide, N-methyl-N-phenylacetamide, N-formylmorpholine, N-acetylmorpholine, N-formylpiperidine, N-acetylpiperidine and N-acetyl-N'-methyl-piperazine and the corresponding thiocompounds. Particularly suitable amides having the formula I are N,N-dimethylacetamide and N-methylpyrrolidone. Formula I also covers diamides and triamides such as diacetamide, triacetamide, dibenzamide, tribenzamide and N-methyldibenzamide and imines such as succinimide, 1,2-cyclohexanedicarboximide and N-phenyl-phthalimide. Usable carbamates have, for example, the formula

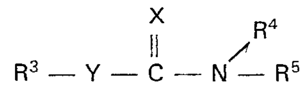 II

in which $R^3$ represents an alkyl, aryl, aralkyl or alkaryl group with not more than 10 carbon atoms, $R^4$ and $R^5$ represent, independently of each other, a hydrogen atom or an alkyl, aryl, aralkyl or alkaryl group with not more than 10 carbon atoms and X and Y represent, independently of each other, oxygen or sulphur. Specific examples of such amides are methyl carbamate, ethyl carbamate, phenyl carbamate, N-methyl methylcarbamate, N-ethyl methylcarbamate, N-phenyl methylcarbamate, N-methyl ethylcarbamate, N-ethyl ethylcarbamate, N-phenyl ethylcarbamate, N-methyl phenylcarbamate, N-ethyl phenylcarbamate, N-phenyl phenylcarbamate, N,N-dimethyl methylcarbamate, N,N-diethyl methyl ethylcarbamate, N,N-diphenyl methylcarbamate, N,N-dimethyl ethylcarbamate, N,N-diethyl ethylcarbamate, N,N-diphenyl ethylcarbamate, N,N-dimethyl phenylcarbamate, N,N-diethyl phenylcarbamate, N,N-diphenyl phenylcarbamate, N-methyl-N-ethyl methylcarbamate, N-methyl-N-ethyl ethylcarbamate and the corresponding thiocompounds.

The urea (derivative) has, for example, the formula

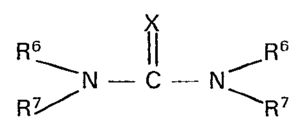 III

in which $R^6$ and $R^7$ represent, independently of each other, a hydrogen atom or alkyl, aryl, aralkyl or alkaryl groups with not more than 10 carbon atoms and X represents oxygen or sulphur. Specific examples of such urea derivatives are urea, thiourea, 1,3-dimethylurea, 1,3-diethylurea, 1,3-diphenylurea, 1,1-dimethylurea, 1,1-diphenylurea and 1,1,3,3-tetramethylurea.

The quantity of organo-nitrogen compound preferably lies between 0.5 and 3 mol, in particular between 0.8 and 2 mol per mol alcohol.

The alcohols used as starting material in the process according to the invention are preferably hydrocarbons comprising 1-20, in particular 1-12, carbon atoms, substituted with one or more hydroxy groups and optionally inert substituents. Examples of suitable alcohols are methanol, ethanol, propanol, isopropanol, butanol, sec.butanol, tert.butanol, the hexanols, the octanols, phenol, benzyl alcohol, ethylene glycol, glycerol and catechol. Alkanols, in particular with 1-12, particularly 1-6, carbon atoms are preferred. The alcohol can also be produced in situ from derivatives which dissociate to form an alcohol under the prevalent reaction conditions. In

this way, alcohols can be produced from ethers by hydrolysis with the water present in the reaction mixture.

The palladium-containing catalyst is preferably a compound soluble in the reaction mixture or a soluble complex of palladium. Examples of suitable palladium compounds are palladium salts such as palladium chloride, palladium bromide, palladium iodide, palladium nitrate, palladium acetate, palladium propionate and palladium isobutyrate. Examples of suitable palladium complexes are palladium acetylacetonate, sodium tetrachloropalladate, potassium tetrachloropalladate, potassium tetraiodopalladate and complexes such as $[Pd(CO)Cl_2]_2$, $Pd[(C_6H_5)_3P]_2I_2$, $[(n\text{-}C_4H_9)_4P]_2\,[PdCl_4]$, $Pd[(C_6H_5)_3P]_2\,(CO)Br$ and $Pd[(n\text{-}C_4H_9)_3P]_2I_2$.

The palladium-containing catalyst is preferably used in a quantity of $10^{-2}$ - $10^{-6}$, in particular $10^{-3}$ - $10^{-5}$ gram atoms palladium per mol alcohol. If desirable, a co-catalyst may be present besides the palladium-containing catalyst. Nickel compounds, in particular nickel salts such as, for example, nickel chloride, are highly suitable co-catalysts. If the process according to the invention is effected in the presence of a nickel compound as co-catalyst, the carboxylic acid is formed at a very high rate. Production rates of more than 300 g carboxylic acid/g Pd/g l/hour can readily be achieved. Such rates are even higher than the rates achieved with the far more expensive rhodium catalysts.

The iodide and/or bromide source may, for example, be elemental iodine or bromine, or hydrogen iodide or bromide, or an iodide or bromide of an alkali metal, alkaline earth metal or transition metal, or a compound $R^8I$, $R^8Br$, $R^8COI$ or $R^8COBr$, in which $R^8$ is an optionally bromine or iodine substituted alkyl group or an aralkyl group with preferably not more than 12 carbon atoms. Examples of suitable iodide and bromide sources are lithium, sodium, potassium, rubidium and caesium iodide, lithium, sodium, potassium, rubidium and caesium bromide, calcium iodide, calcium bromide, magnesium iodide, magnesium bromide and the iodides and bromides of transition metals such as copper, zinc vanadium, chromium, manganese and nickel. If the palladium or a metal acting as co-catalyst is present in iodide or bromide form in the catalyst, it can equally act as iodide or bromide source. Lithium iodide and sodium iodide are highly suitable iodide sources, as are $R^8I$ and $R^8COI$ compounds in which $R^8$ represents an alkyl group with 1-4 carbon atoms, in particular methyl iodide. If desirable, more than one of said compounds may be used as iodide source. Other specific examples of suitable iodide and/or bromide sources with the formula $R^8I$ or $R^8Br$ are $CH_3Br$, $C_2H_5I$, $C_4H_9I$, $C_8H_{17}I$, $CH_2I_2$, $C_2H_4I_2$, $CH_2IBr$, $CHI_3$ and $C_2H_4IBr$. Ammonium, phosphonium, arsonium and stibonium iodides and bromides can likewise be used as iodide and/or bromide source. Examples of such compounds are triphenylphosphonium iodide, methyltriphenylphosphonium iodide, tetramethylammonium iodide, tetraisopropylammonium iodide, methyltriphenylphosphonium bromide, tetrabutylammonium bromide and tetrabutylammonium iodide.

The quantity of iodide and/or bromide source, i.e. the total number of gram atoms I and/or Br present in the reaction mixture, lies generally between 1 and 1000, preferably between 3 and 500 and particularly between 10 and 300 gram atoms I and/or Br per gram atom palladium.

The reaction is preferably effected in the presence of an additional promoter. Said additional promoter is a compound with the formula

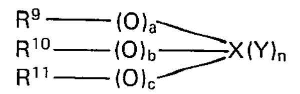

IV

in which X represents nitrogen, phosphorus, arsenic or antimony and Y oxygen, sulphur or selenium, n is 0 or 1 and either a, b and c are 0 or 1 and $R^9$, $R^{10}$ and $R^{11}$ represent, independently of each other, optionally substituted hydrocarbon groups, or a and b are 0, c is 0 or 1, $R^9$ and $R^{10}$ together with X form a heterocyclic group and $R^{11}$ represents an optionally substituted hydrocarbon group, or a, b and c are 0 and $R^9$, $R^{10}$ and $R^{11}$ together with X form a heterocyclic aromatic group, or $R^9$ is hydrogen and $R^{10}$ and $R^{11}$ represent an optionally substituted hydrocarbon group.

The hydrocarbon groups represented by $R^9$, $R^{10}$ and $R^{11}$ may be alkyl, cycloalkyl, aryl, aralkyl or alkaryl groups with preferably not more than 30, particularly not more than 20, carbon atoms and are optionally substituted with one or more substituents, such as, for example, halogen atoms or an $R^{12}R^{13}X(Y)_n$ group in which X, Y and n have the hereinbefore stated meanings and $R^{12}$ and $R^{13}$ each represent an optionally substituted hydrocarbon group. If $R^9$ and $R^{10}$ with X form a heterocyclic group, said group preferably contains not more than 20 carbon atoms. Specific examples of suitable heterocyclic groups are the phospholane, phosphorinane and phosphorus heptane groups in which the groups $R^9$ and $R^{10}$ together represent an alkylene group with 4, 5 or 6 carbon atoms respectively, and the 9-phosphabicyclo [4.2.1] nonane and the 9-phosphabicyclo [3.3.1] nonane groups. Said heterocyclic groups may, for example, be substituted with hydrocarbon groups.

Formula IV compounds in which a, b and c are 0, X is phosphorus, arsenic or antimony, Y is oxygen or sulphur and n is 0 or 1 and $R^9$, $R^{10}$ and $R^{11}$ represent alkyl groups with 1-12 carbon atoms or cycloalkyl, aryl, aralkyl or alkaryl groups with not more than 12 carbon atoms, constitute a preferred group of promoters. Particular preference is given to formula IV compounds in which a, b and c are 0, X is phosphorus, Y oxygen and n is 0 or 1 and $R^9$, $R^{10}$ and $R^{11}$ represent, independently of each other, alkyl groups with 1-12 carbon atoms or phenyl groups optionally substituted with 1 or 2 methyl or ethyl groups.

Examples of formula IV compounds in which X represents phosphorus, arsenic or antimony and a, b and c are 0 are secondary and tertiary phosphines, arsines and stibines such as, for example, trimethylphosphine, diethylphosphine, triethylphosphine, tri-n-butylphosphine, trioctylphosphine, diphenylphosphine, triphenylphosphine, tri-p-tolylphosphine, tricyclohexylphosphine, diphenylethylphosphine, tri-(1-naphthyl)-phosphine, tri-4-chlorophenylphosphine, triethylarsine, triphenylarsine and triphenylstibine and the oxides, sulphides and selenides of said compounds. Phosphines and phosphine oxides, in particular triphenylphosphine and triphenylphosphine oxide, are preferred promoters.

Examples of compounds with the general formula IV containing a heterocyclic group are 1-phenylphospholane, 1-phenylphosphorinane, 9-phenyl-9-phosphabicyclo [4.2.1] nonane, 9-phenyl-9-phosphabicyclo [3.3.1] nonane, 9-eicosyl-9-phosphabicyclo [4.2.1] and 9-eicosyl-9-phosphabicyclo [3.3.1] nonane and the oxides, sulphides and selenides of said compounds.

Formula IV compounds in which a and/or b and/or c are 1 and n is 1 are the alkyl, cycloalkyl, aryl, aralkyl or alkaryl esters of phosphoric acid, phosphonic acids and phosphinic acids and the analogues of said compounds in which the doubly bonded oxygen atom is replaced by a sulphur or selenium atom and/or the phosphorus atom by an arsenic or antimony atom. Specific examples of such compounds are trimethyl phosphate, tri-n-butyl phosphate, triphenyl phosphate, dimethylmethyl phosphonate, diethylmethyl phosphonate, diphenylmethyl phosphonate, methyldiethyl phosphinate and phenyldimethyl phosphinate.

Examples of formula IV compounds in which X is nitrogen are tertiary amines such as trimethylamine, triethylamine, N,N-dimethylphenylamine, N,N-dimethyl-p-methylphenylamine, N-methyl-piperidine, N,N-dimethyloctylamine and N,N-dimethyldodecylamine, and heterocyclic amines such as, for example, pyrrole, pyrrolidine, pyridine, piperidine, pyrimidine, pyrazine, benzotriazole and morpholine and the oxides thereof. The heterocyclic amines or their oxides may optionally be substituted with one or more alkyl groups, in particular methyl groups. Pyridine and the alkyl-substituted pyridines such as, for example, the picolines, in particular alpha-picoline and the oxides thereof, are highly suitable additional promoters.

Examples of formula IV compounds in which one or more of the $R^9$, $R^{10}$ and $R^{11}$ groups are substituted with an $R^{12}R^{13}X(Y)_n$ group are N,N,N',N'-tetramethylethylene diamine, N,N,N',N'-tetramethylethylenediamine-N,N'-dioxide, tetraphenyldiphosphinoethane, tetraphenyldiphosphinopropane, tetraphenyldiphosphinoethane dioxide, tetraphenyldiphosphinopropane dioxide, diphenylditolyldiphosphinoethane and 1,2-bis(diphenylphosphino)-benzene, 2,2'-bipyridine and 2,2'-biquinoline. Examples of compounds with general formula IV in which a, b and c are 0 and $R^9$, $R^{10}$, $R^{11}$ together with X form a heterocyclic group are 1,10-phenanthroline, 1,10-phenanthroline-N,N'-dioxide, pyrazine and pyrazine-N,N'-dioxide.

In the process according to the invention, complexes obtained by reacting a formula IV compound with a hydrocarbon iodide or bromide such as, for example, $CH_3I$, an acyl iodide or bromide, or hydrogen iodide or bromide can also be used as iodide or bromide source. Examples of such complexes are: $[(C_6H_5)_3PO\text{-}H\text{-}OP(C_6H_5)_3]^+I_3^-$ and $[(C_2H_5)_3AsO\text{-}H\text{-}OAs(C_2H_5)_3]^+I^-$. Such complexes are probably also produced in situ by reacting a formula IV compound and iodine or bromine compounds present in the reaction mixture.

Formula IV compounds in which X represents phosphorus and Y oxygen, n is 1 and a, b and c are 0 can be generated in situ by adding the corresponding phosphine to the reaction mixture and by effecting the reaction in the presence of molecular oxygen or hydrogen peroxide.

The quantity of formula IV compound used as promoter in the process according to the invention can lie, for example, between 0.1 and 300 mol per gram atom, preferably between 1 and 200 and in particular between 10 and 150 mol per gram atom palladium.

In the process according to the invention, particularly high production rates in terms of the carboxylic acid can be achieved by effecting the reaction in the presence of a catalyst system containing both palladium and nickel, a formula I organo-nitrogen compound, in particular N-methylpyrrolidone, an alkali metal iodide and a phosphine oxide. Furthermore, high production rates can be achieved by using a catalyst system containing palladium, a formula I organo-nitrogen compound, an alkyliodide, in particular methyl iodide, an alkali metal iodide and a phosphine oxide.

The process according to the invention is effected at a temperature preferably between 110 and 225°C, in particular between 125 and 200°C. The reaction is generally effected at a partial carbon monoxide pressure between 0.5 and 70 bar. High pressures up to, for example, 1000 bar may be used if desirable, but are not generally attractive for technical and economic reasons.

The carbon monoxide used in the process according to the invention may optionally be mixed with other gases such as, for example, hydrogen, carbon dioxide, methane, nitrogen or noble gases. Carbon monoxide or a carbon monoxide-containing gas containing less than 50%v hydrogen is preferred.

The process is effected in the liquid phase. The use of an (additional) solvent is not usually necessary because the alcohol used as starting material and the organo-nitrogen-compound act as solvents to a sufficient extent. Other reaction mixtures constituents, for example a liquid iodide source, such as, for example, $CH_3I$, or the ester or carboxylic acid formed may also act as solvents. If required, additional quantities of those compounds may be added to the reaction mixture. Suitable (additional) solvents are, for example, acetic acid, propionic acid, methyl acetate, butyrolactone, dimethyl ether, diethyl ether, acetic anhydride, methyl-t.butyl ether, diglym, tetraglym, tetrahydrofuran, 1,4-dioxane, 1,3-dioxane, dimethyl sulphone, diethyl sulphone, methylethyl sulphone, methylbutyl sulphone, sulfolane, 2-methylsulfolane, 3-methylsulfolane, 2-methyl-4-butylsulfolane, dimethyl sulphoxide and diethyl sulphoxide. Water may also be present, as a result of which formation of the carboxylic acid at the expense of the ester is generally promoted.

The process according to the invention may be effected continuously, semi-continuously or batchwise. The reaction mixture obtained may be further processed by means of known techniques such as, for example, fractionation. Moreover, the process can be integrated with existing processes for the preparation of the starting materials or for the further processing of the carboxylic acid or ester obtained.

*Examples*

A 300 ml magnetically stirred Hastelloy C autoclave (Hastelloy is a trade name) was charged with 0.1 mol palladium acetate and the quantities of methanol, organo-nitrogen compound, $NiCl_2 \cdot 6H_2O$, methanol, alkali metal iodide and additional promoter stated in the Table. The autoclave was flushed with carbon monoxide and filled with a carbon monoxide and hydrogen mixture. The partial pressures of the carbon monoxide and the hydrogen measured 30 and 15 bar respectively. The autoclave was heated to 170°C for the duration of the reaction time stated in the Table and subsequently cooled. The contents of the autoclave were analysed by means of gas/liquid chromatography. The quantities of acetic acid and methyl acetate were determined and the total quantity of acetic acid formed by methanol carbonylation calculated. The carbonylation rate, expressed as g acetic acid/g Pd/g l/hour and, where a nickel compound was present as co-catalyst, as g acetic acid/g Ni/g l/hour is stated in the Table.

The Table shows that where less than 0.3 mol of the organo-nitrogen compound to be used in accordance with the invention is present per mol methanol, virtually no acetic acid is formed. It is also evident that where the organo-nitrogen compound to be used in accordance with the invention is replaced by an amine such as pyridine in a quantity of more than 0.3 mol per mol methanol, no acetic acid is produced. Finally, the tests show that by adding a nickel compound as co-catalyst, despite the application of moderate reaction conditions, acetic acid is produced at a very high rate.

| Test No. | Methanol (mol) | $NiCl_2 \cdot 6H_2O$ (mmol) | Organo-nitrogen compound (mol) | | Methyl-iodide (mmol) | Alkali metal iodide (mmol) | |
|---|---|---|---|---|---|---|---|
| 1 | 0.25 | — | NMP*) | 0.36 | 30 | — | |
| 2 | 0.25 | — | NMP | 0.36 | 12.5 | $LiI \cdot 2H_2O$ | 18 |
| 3 | 0.25 | 2.4 | NMP | 0.36 | 12.5 | $LiI \cdot 2H_2O$ | 18 |
| 4 | 0.25 | 2.4 | NMP | 0.36 | 30 | — | |
| 5 | 0.25 | 2.4 | NMP | 0.36 | — | $LiI \cdot 2H_2O$ | 18 |
| 6 | 0.25 | — | DMA***) | 0.37 | 12.5 | $LiI \cdot 2H_2O$ | 18 |
| 7 | 0.25 | — | NMP | 0.36 | 12.5 | NaI | 18 |
| 8 | 0.25 | 2.4 | NMP | 0.36 | 30 | — | |
| 9°) | 0.65 | 1 | NMP | 0.36 | 25 | $LiI \cdot 2H_2O$ | 36 |
| | | | Comparative tests | | | | |
| 10 | 0.25 | — | Pyridine | 0.35 | 12.5 | $LiI \cdot 2H_2O$ | 18 |
| 11 | 0.50 | — | NMP | 0.1 | 12.5 | $LiI \cdot 2H_2O$ | 18 |
| 12 | 1.75 | — | NMP | 0.36 | 12.5 | $LiI \cdot 2H_2O$ | 18 |

*) NMP = N-methylpyrrolidone ***) DMA = N,N-dimethylacetamide

°) In this test the temperature was 155°C; partial pressures of CO and $H_2$ were 26.6 and 14.4 bar respectively.

| Additional promoter (mmol) | | Reaction time (hours) | Conversion to carbonylated product % | g acetic acid/g Pd/g l/h | g acetic acid/g Ni/g l/h |
|---|---|---|---|---|---|
| — | | 2 | 28.6 | 53 | |
| PO**) | 12 | 1.5 | 52.3 | 127 | |
| PO | 12 | 1.5 | 96 | 233 | 18 |
| PO | 12 | 1.5 | 81 | 200 | 15 |
| PO | 12 | 1.5 | 81 | 333 | 25 |
| PO | 12 | 1.5 | 31 | 75 | |
| PO | 12 | 1 | 52.7 | 190 | |
| — | | 1.5 | 74.7 | 185 | 14 |
| PO | 12 | 1 | 30.6 | 145 | 26 |

*(continued)*

| Additional promoter (mmol) | Reaction time (hours) | Conversion to carbonylated product % | g acetic acid/g Pd/g l/h | g acetic acid/g Ni/g l/h |
|---|---|---|---|---|
| PO 12 | 2 | 0 | — | |
| PO 12 | 2 | 1.3[xxxx] | 4.7 | |
| PO 12 | 2 | < 0.3[xxxx] | 4.1 | |

[xx] PO = triphenylphosphine oxide
[xxxx] Reaction ceased after 1 hour.

## Claims

1. Process for the preparation of carboxylic acids and/or esters by reacting an alcohol with carbon monoxide in the presence of a homogeneous palladium-containing catalyst, an iodide and/or bromide source and an organo-nitrogen compound, characterized in that an organo-nitrogen compound containing an 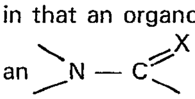 group (X being oxygen or sulphur) in a quantity of at least 0.3 mol per mol alcohol is used as promoter.

2. Process as claimed in claim 1, characterized in that the organo-nitrogen compound is an amide, carbamate or urea (derivative) that is liquid at the temperature and pressure applied in the course of the reaction.

3. Process as claimed in claim 2, characterized in that the amide has the formula

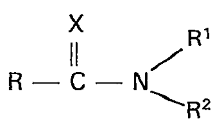 I

in which R represents a hydrogen atom or an alkyl, cycloalkyl, aryl, alkaryl or aralkyl group with not more than 10 carbon atoms, $R^1$ and $R^2$ represent, independently of each other, a hydrogen atom or an alkyl, aryl, alkaryl or aralkyl group, which may contain a 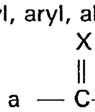-group, with not more than 10 carbon atoms or $R^1$ and $R^2$ together with the nitrogen atom to which they are bonded form a cyclic group with not more than 5 carbon atoms, which may contain one or more nitrogen or oxygen atoms, or one of the groups $R^1$ and $R^2$ together with the nitrogen atom and R forms a cyclic group with not more than 5 carbon atoms, and X represents oxygen or sulphur.

4. Process as claimed in claim 2 or 3, characterized in that the amide is N,N-dimethylacetamide or N-methylpyrrolidone.

5. Process as claimed in claim 2, characterized in that the carbamate has the formula

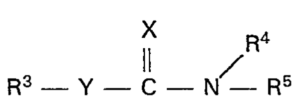

in which $R^3$ represents an alkyl, aryl, aralkyl or alkaryl group with not more than 10 carbon atoms, $R^4$ and $R^5$ represent, independently of each other, a hydrogen atom or an alkyl, aryl, aralkyl or alkaryl group with not more than 10 carbon atoms and X and Y represent, independently of each other, oxygen or sulphur.

6. Process as claimed in claim 2, characterized in that the urea (derivative) has the formula

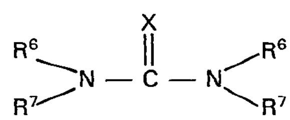

in which $R^6$ and $R^7$ represent, independently of each other, a hydrogen atom or alkyl, aryl, aralkyl or alkaryl groups with not more than 10 carbon atoms and X represents oxygen or sulphur.

7. Process as claimed in claims 1-6, characterized in that the quantity of organo-nitrogen compound lies between 0.5 and 3, in particular between 0.8 and 2 mol per mol alcohol.

8. Process as claimed in claims 1-7, characterized in that the alcohol is a hydrocarbon, substituted with one ore more hydroxy groups, with 1-20 carbon atoms, in particular an alkanol with 1-12 carbon atoms.

9. Process as claimed in claims 1-8, characterized in that the iodide and/or bromide source is elemental iodine or bromine, or hydrogen iodide or bromide, an iodide or bromide of an alkali metal, alkaline earth metal or transition metal, or an $R^8I$, $R^8Br$, $R^8COI$ or $R^8COBr$ compound, in which $R^8$ is an optionally bromine or iodine substituted alkyl group or an aralkyl group with preferably not more than 12 carbon atoms.

10. Process as claimed in claim 9, characterized in that one or more of the compounds lithium iodide, sodium iodide and $R^8I$ or $R^8COI$ compounds, in which $R^8$ is an alkyl group with 1-4 carbon atoms, in particular methyl iodide, is used as iodide source.

11. Process as claimed in claims 1-10, characterized in that the reaction is effected in the presence of an additional promoter with the formula

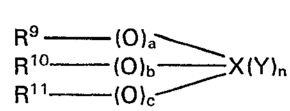

in which X represents nitrogen, phosphorus, arsenic or antimony and Y oxygen, sulphur or selenium, n is 0 or 1 and either a, b and c are 0 or 1 and $R^9$, $R^{10}$

and $R^{11}$ represent, independently of each other, optionally substituted hydrocarbon groups, or a and b are 0, c is 0 or 1, $R^9$ and $R^{10}$ together with X form a heterocyclic group and $R^{11}$ represents an optionally substituted hydrocarbon group, or a, b and c are 0 and $R^9$, $R^{10}$ and $R^{11}$ together with X form a heterocyclic aromatic group, or $R^9$ is hydrogen and $R^{10}$ and $R^{11}$ represent an optionally substituted hydrocarbon group.

12. Process as claimed in claim 11, characterized in that a, b and c are 0, X is phosphorus, Y oxygen and n is 0 or 1 and $R^9$, $R^{10}$ and $R^{11}$ represent, independently of each other, alkyl groups with 1-12 carbon atoms or phenyl groups optionally substituted with 1 or 2 methyl or ethyl groups.

13. Process as claimed in claims 1-12, characterized in that the reaction is effected in the presence of a nickel compound as co-catalyst.

**Patentansprüche**

1. Verfahren zur Herstellung von Carbonsäuren und/oder -estern durch Umsetzung eines Alkohols mit Kohlenmonoxid in Gegenwart eines homogenen Palladium enthaltenden Katalysators einer Iodid- und/oder Bromidquelle und einer organischen Stickstoffverbindung, dadurch gekennzeichnet, dass eine organische Stickstoffverbindung, enthaltend eine Gruppe 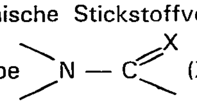 (X gleich Sauerstoff oder Schwefel) in einer Menge von mindestens 0,3 Mol pro Mol Alkohol als Promotor verwendet wird.

2. Verfahren nach Anspruch 1, dadurch gekennzeichnet, dass die Organo-Stickstoff-Verbindung ein Amid, Carbamat oder Harnstoff (-Derivat) ist, die bei den im Verlauf der Reaktion angewandten Temperatur- und Druckbedingungen flüssig ist.

3. Verfahren nach Anspruch 2, dadurch gekennzeichnet, dass das Amid die Formel

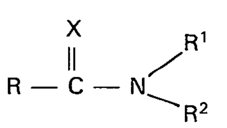

besitzt, in der R ein Wasserstoffatom oder eine Alkyl-, Cycloalkyl-, Aryl-, Alkaryl- oder Aralkylgruppe mit nicht mehr als 10 Kohlenstoffatomen ist, $R^1$ und $R^2$ unabhängig voneinander ein Wasserstoffatom oder eine Alkyl-, Aryl-, Alkaryl- oder Aralkylgruppe mit nicht mehr als 10 Kohlenstoffatomen, die eine Gruppe 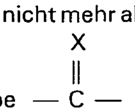 enthalten können, bedeutet, oder $R^1$ und $R^2$ zusammen mit dem Stickstoffatom, an das sie gebunden sind, eine cyclische Gruppe mit nicht mehr als 5 Kohlenstoffatomen bilden, die ein oder mehrere Stickstoff- oder Sauerstoffatome enthalten kann, oder eine der Gruppen $R^1$ und $R^2$ zusammen mit dem Stickstoffatom und R eine cyclische Gruppe mit nicht mehr als 5 Kohlenstoffatomen bilden, und X Sauerstoff oder Schwefel bedeutet.

4. Verfahren nach Anspruch 2 oder 3, dadurch gekennzeichnet, dass das Amid N,N-Dimethylacetamid oder N-Methylpyrrolidon ist.

5. Verfahren nach Anspruch 2, dadurch gekennzeichnet, dass das Carbamat die Formel

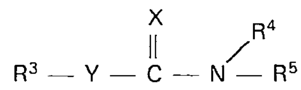

besitzt, in der $R^3$ eine Alkyl-, Aryl-, Aralkyl- oder Alkarylgruppe mit nicht mehr als 10 Kohlenstoffatomen bedeutet, $R^4$ und $R^5$ unabhängig voneinander ein Wasserstoffatom oder eine Alkyl-, Aryl-, Aralkyl- oder Alkarylgruppe mit nicht mehr als 10 Kohlenstoffatomen bedeuten, und X und Y unabhängig voneinander Sauerstoff oder Schwefel sind.

6. Verfahren nach Anspruch 2, dadurch gekennzeichnet, dass der Harnstoff (das Harnstoffderivat) die Formel

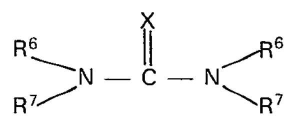

besitzt, in der $R^6$ und $R^7$ unabhängig voneinander ein Wasserstoffatom oder Alkyl-, Aryl-, Aralkyl- oder Alkarylgruppen mit nicht mehr als 10 Kohlenstoffatomen bedeuten und X Sauerstoff oder Schwefel ist.

7. Verfahren nach den Ansprüchen 1 bis 6, dadurch gekennzeichnet, dass die Menge an Organo-Stickstoff-Verbindung zwischen 0,5 und 3, insbesondere zwischen 0,8 und 2 Mol pro Mol Alkohol liegt.

8. Verfahren nach den Ansprüchen 1 bis 7, dadurch gekennzeichnet, dass der Alkohol ein durch eine oder mehrere Hydroxygruppen substituierter Kohlenwasserstoff mit 1 bis 20 Kohlenstoffatomen, insbesondere ein Alkanol mit 1 bis 12 Kohlenstoffatomen ist

9. Verfahren nach den Ansprüchen 1 bis 8, dadurch gekennzeichnet, dass die Iodid- und/oder Bromidquelle elementares Iod oder Brom oder Iodwasserstoff oder Bromwasserstoff, ein Alkali-, Erdalkali- oder Übergangsmetalliodid oder -bromid ist oder eine Verbindung $R^8I$, $R^8Br$, $R^8COI$ oder $R^8COBr$, in der $R^8$ eine, gegebenenfalls durch Brom oder Iod substituierte, Alkylgruppe oder eine Aralkylgruppe mit vorzugsweise nicht mehr als 12 Kohlenstoffatomen ist.

10. Verfahren nach Anspruch 9, dadurch gekennzeichnet, dass eine oder mehrere der Verbindungen Lithiumiodid, Natriumiodid und $R^8I$ oder $R^8COI$, bei denen $R^8$ eine Alkylgruppe mit 1 bis 4 Kohlenstoffatomen ist, insbesondere Methyliodid als Iodidquelle verwendet wird.

11. Verfahren nach den Ansprüchen 1 bis 10, dadurch gekennzeichnet, dass die Reaktion durchgeführt wird in Gegenwart eines zusätzlichen Promotors der Formel

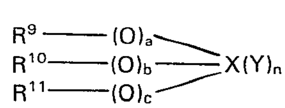

in der X Stickstoff, Phosphor, Arsen oder Antimon bedeutet, Y Sauerstoff, Schwefel oder Selen ist, n 0 oder 1 ist, und entweder a, b und c 0 oder 1 sind, und $R^9$, $R^{10}$ und $R^{11}$ unabhängig voneinander gegebenenfalls substituierte Kohlenwasserstoffgruppen bilden, oder a und b 0 und c 0 oder 1 ist, und $R^9$ und $R^{10}$ zusammen mit X eine heterocyclische Gruppe bedeuten, und $R^{11}$ eine gegebenenfalls substituierte Kohlenwasserstoffgruppe ist, oder a, b und c 0 sind, und $R^9$, $R^{10}$ und $R^{11}$ zusammen mit X eine heterocyclische aromatische Gruppe bilden, oder $R^9$ Wasserstoff ist und $R^{10}$ und $R^{11}$ eine gegebenenfalls substituierte Kohlenwasserstoffgruppe sind.

12. Verfahren nach Anspruch 11, dadurch gekennzeichnet, dass a, b und c 0 sind, X Phosphor und Y Sauerstoff ist, und n 0 oder 1 ist und $R^9$, $R^{10}$ und $R^{11}$ unabhängig voneinander Alkylgruppen mit 1 bis 12 Kohlenstoffatomen oder Phenylgruppen, gegebenenfalls substituiert durch ein oder zwei Ethyl- oder Methylgruppen, sind.

13. Verfahren nach den Ansprüchen 1 bis 12, dadurch gekennzeichnet, dass die Reaktion in Gegenwart einer Nickelverbindung als Co-Katalysator durchgeführt wird.

**Revendications**

1. Procédé pour la préparation d'acides carboxyliques et/ou d'esters par réaction d'un alcool avec l'oxyde de carbone en présence d'un catalyseur homogène contenant du palladium, d'une source d'iodure et/ou de bromure et d'un composé organo-azote, caractérisé en ce qu'on utilise comme promoteur un composé organo-azote contenant un groupe 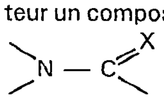 (X étant de l'oxygène ou du soufre) à raison d'au moins 0,3 mole par mole d'alcool.

2. Procédé selon la revendication 1, caractérisé en ce que le composé organo-azote est un amide, un carbamate ou un dérivé d'urée qui est liquide à la température et à la pression utilisées au cours de la réaction.

3. Procédé selon la revendication 2, caractérisé en ce que l'amide a la formule

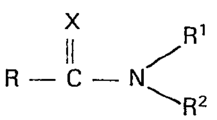

où X représente un atome d'hydrogène ou un groupe alcoyle, cycloalcoyle, aryle, alcaryle ou aralcoyle n'ayant pas plus de 10 atomes de carbone, $R^1$ et $R^2$ représentent, indépendamment l'un de l'autre, un atome d'hydrogène ou un groupe alcoyle, aryle, alcaryle ou aralcoyle, qui peut contenir un groupe 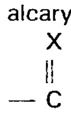, n'ayant pas plus de 10 atomes de carbone ou $R^1$ et $R^2$ en même temps que l'atome d'azote auquel ils sont liés forment un groupe cyclique n'ayant pas plus de 5 atomes de carbone, qui peut contenir un ou plusieurs atomes d'azote ou d'oxygène, ou un des groupes $R^1$ et $R^2$ en même temps que l'atome d'azote et R forme un groupe cyclique n'ayant pas plus de 5 atomes de carbone, et X représente de l'oxygène ou du soufre.

4. Procédé selon la revendication 1 ou 2, caractérisé en ce que l'amide est le N,N-diméthylacétamide ou la N-méthylpyrrolidone.

5. Procédé selon la revendication 2, caractérisé en ce que le carbamate a la formule

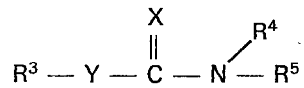

dans laquelle $R^3$ représente un groupe alcoyle, aryle, aralcoyle ou alcaryle n'ayant pas plus de 10 atomes de carbone, $R^4$ et $R^5$ représentent, indépendamment l'un de l'autre, un atome d'hydrogène ou un groupe alcoyle, aryle, aralcoyle ou alcaryle n'ayant pas plus de 10 atomes de carbone et X et Y représentent, indépendamment l'un de l'autre, de l'oxygène ou du soufre.

6. Procédé selon la revendication 2, caractérisé en ce que le dérivé d'urée a la formule

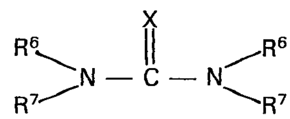

dans laquelle $R^6$ et $R^7$ représentent, indépendamment l'un de l'autre, un atome d'hydrogène ou des groupes alcoyle, aryle, aralcoyle ou alcaryle n'ayant pas plus de 10 atomes de carbone et X est de l'oxygène ou du soufre.

7. Procédé selon l'une quelconque des revendications 1 à 6, caractérisé en ce que la quantité de composé organo-azote est comprise entre 0,5 et 3, en particulier entre 0,8 et 2 moles par mole d'alcool.

8. Procédé selon l'une quelconque des revendications 1 à 7, caractérisé en ce que l'alcool est un hydrocarbure substitué par un ou plusieurs groupes hydroxy, ayant 1-20 atomes de carbone, en particulier un alcanol ayant 1-12 atomes de carbone.

9. Procédé selon l'une quelconque des revendications 1 à 8, caractérisé en ce que la source d'iodure et/ou de bromure est de l'iode ou du brome élémentaire, de l'iodure ou du bromure d'hydrogène, un iodure ou bromure d'un métal alcalin, d'un métal alcalino-terreux ou d'un métal de transition ou un composé $R^8I$, $R^8Br$, $R^8COI$ ou $R^8COBr$, où $R^8$ est un groupe alcoyle éventuellement bromé ou iodé ou un groupe aralcoyle n'ayant de préférence pas plus de 12 atomes de carbone.

10. Procédé selon la revendication 9, caractérisé en ce que comme source d'iodure on utilise un ou plusieurs des composés iodure de lithium, iodure de sodium, $R^8I$ ou $R^8COI$, où $R^8$ est un groupe alcoyle de 1-4 atomes de carbone, en particulier l'iodure de méthyle.

11. Procédé selon l'une quelconque des revendications 1 à 10, caractérisé en ce que la réaction est conduite en présence d'un promoteur supplémentaire de la formule

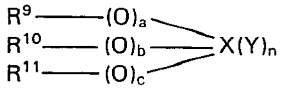

dans laquelle X représente de l'azote, du phosphore, de l'arsenic ou de l'antimoine et Y de l'oxygène, du soufre ou du sélénium, n est 0 ou 1 et ou bien a, b et c sont 0 ou 1 et $R^9$, $R^{10}$ et $R^{11}$ représentent, indépendamment les uns des autres, des groupes d'hydrocarbures éventuellement substitués, ou bien a et b sont 0, c est 0 ou 1, $R^9$ et $R^{10}$ en même temps que X forment un groupe hétérocyclique et $R^{11}$ représente un groupe d'hydrocarbure éventuellement substitué ou encore a, b et c sont 0 et $R^9$, $R^{10}$ et $R^{11}$ en même temps que X forment un groupe aromatique hétérocyclique, ou $R^9$ est de l'hydrogène et $R^{10}$ et $R^{11}$ représentent un groupe d'hydrocarbure éventuellement substitué.

12. Procédé selon la revendication 11, caractérisé en ce que a, b et c sont 0, X est du phosphore, Y de l'oxygène et n est 0 ou 1 et $R^9$, $R^{10}$ et $R^{11}$ représentent, indépendamment les uns des autres, des groupes alcoyle de 1-12 atomes de carbone ou des groupes phényle éventuellement substitués par 1 ou 2 groupes méthyle ou éthyle.

13. Procédé selon l'une quelconque des revendications 1 à 12, caractérisé en ce que la réaction est conduite en présence d'un composé du nickel comme co-catalyseur.